# EUROPÄISCHE PATENTSCHRIFT

(11) **EP 2 022 801 B1**
(45) Veröffentlichungstag und Bekanntmachung des Hinweises auf die Patenterteilung: **17.01.2018**
(21) Anmeldenummer: 07015804.3
(22) Anmeldetag: 10.08.2007
(51) Int. Cl.: C08B 15/02

(54) **Nanopartikel aus amorpher Cellulose**
Nanoparticles made of amorphous cellulose
Nanoparticules en cellulose amorphe

(43) Veröffentlichungstag der Anmeldung: 11.02.2009
(73) Patentinhaber: Dow Global Technologies LLC, Midland, MI 48674 (US)
(72) Erfinder: Nachtkamp, Klaus, 29664 Walsrode (DE); Krüger, Christa, 29640 Schneverdingen (DE); Engelhardt, Jürgen, 29683 Bad Fallingbostel (DE); Fischer, Steffen, 01705 Freital (DE); Pinnow, Manfred, 14514 Teltow (DE); Hettrich, Kay, 14548 Schwielowsee / Caputh (DE)
(74) Vertreter: f & e patent

(56) Entgegenhaltungen:
- EP-A1- 1 036 799
- EP-A1- 1 577 361
- WO-A-2006/034837
- JP-A- 2000 026 229
- JP-A- 2001 192 645
- US-A- 429 534
- US-A- 3 397 198
- US-A- 5 417 984
- US-A1- 2011 259 537
- US-B1- 6 534 071
- DATABASE WPI Week 200454 Derwent Publications Ltd., London, GB; AN 2004-554533 XP002452555 -& CN 1 470 552 A (UNIV DONGHUA) 28. Januar 2004 (2004-01-28)
- ZHANG ET AL: "Facile synthesis of spherical cellulose nanoparticles" CARBOHYDRATE POLYMERS, APPLIED SCIENCE PUBLISHERS, LTD. BARKING, GB, Bd. 69, Nr. 3, 10. Mai 2007 (2007-05-10), Seiten 607-611, XP022068683 ISSN: 0144-8617
- KIM ET AL: "Structural studies of electrospun cellulose nanofibers" POLYMER, ELSEVIER, OXFORD, GB, Bd. 47, Nr. 14, 28. Juni 2006 (2006-06-28), Seiten 5097-5107, XP005526422 ISSN: 0032-3861
- Römpp 3.0: Kolloide
- "A GUIDEBOOK TO PARTICLE SIZE ANALYSIS", Horiba, 1 January 2014 (2014-01-01), XP055240722, Retrieved from the Internet: URL:https://www.horiba.com/fileadmin/uploa ds/Scientific/eMag/PSA/Guidebook/pdf/PSA_G uidebook.pdf [retrieved on 2016-01-13]
- Römpp: Kolloidchemie

## Beschreibung

Die vorliegende Erfindung betrifft neuartige nanoskalige Cellulosepartikel aus amorpher Cellulose sowie ein Verfahren zu deren Herstellung und die Verwendung.

Die Nanotechnologie wird neben der Informations- und Biotechnologie als die große technische Entwicklung unserer Gegenwart angesehen. Ganz allgemein befasst sich die Nanotechnologie mit dem Aufbau, den Eigenschaften und der Wirkung von Strukturen im Bereich von einigen hundert Nanometern (nm) und darunter. Anwendungen entstehen nahezu in allen Bereichen des täglichen Lebens, wie z. B. in der Energie- und Umwelttechnik, in der Informationstechnik sowie im pharmazeutischen und medizinischen Bereich.

Cellulose ist das am häufigsten vorkommende Biopolymer auf der Erde und damit der global bedeutendste nachwachsende Rohstoff. Als Hauptbestandteil der pflanzlichen Gerüstsubstanz weist Cellulose hervorragende molekulare Eigenschaften auf. Bereits im nativen Zustand liegen geordnete Bereiche (Kristallite) mit den typischen Abmessungen von Nanopartikeln (3-10 nm breit und bis zu 100 nm lang) vor. Diese Bereiche sind allerdings verbunden durch nichtkristalline Makromoleküle sowie durch Nebenvalenzbindungen (H-Brücken).

Zur Herstellung von cellulosebasierten Nanopartikeln, welche weitestgehend frei von Überstrukturen sind, sind verschiedene Ansätze bisher verfolgt worden. Allen ist die Überlegung gemein, einzelne Cellulosepartikel voneinander zu separieren und zu stabilisieren, um zu primärpartikulär vorliegende Teilchen zu gelangen, welche nicht durch harte irreversible Agglomeration miteinander verbunden sind.

Üblicherweise werden dazu mechanische und/oder chemische Verfahrensschritte durchlaufen (De Souza Lima, Borsali, Macromol. Rapid Commun. 25 (2004) 771, Ono, Shimaya, Hongo, Yamane, Transactions of the Materials Research Society of Japan 26 (2001) 569, Ioelovich, Leykin, Cellulose Chem. Technol. 40 (2006) 313, Zhang, Elder, Pu, Ragauskas, Carbohydr. Polym. 69 (2007) 607, US-A 2005 0239744, WO 2006/034837 A2, EP 1582551 A1, DE 3047351 C2).

Aus CN 1470552 ist die Herstellung von Cellulosepartikeln in einer Größenordnung von 50 bis 200 nm bekannt, wobei die Cellulose zunächst in einem geeigneten Lösungsmittel gelöst und anschließend durch intensives Rühren in eine Sedimentationslösung dispergiert wird. Zur Stabilisierung der dabei sich bildenden Partikel ist der Zusatz von externen Emulgatoren wie Fettsäuresalzen oder Alkylbenzolsulfonaten notwendig. Nach diesem Verfahren sind nur extrem verdünnte Dispersionen mit einem Cellulosegehalt von unterhalb 0,5 Gew.-% erhältlich.

Die Ergebnisse dieser Methoden sind jedoch hinsichtlich Feinteiligkeit und Verfahrensaufwand nicht befriedigend. Viele der in der Literatur beschriebenen Verfahren führen nämlich zu fibrillären Partikeln, die nur im Querschnitt nanoskalig sind und Faserlängen deutlich über 300 nm besitzen. Bisher ließen sich nanoskalige Partikel nur durch sehr aufwendige Verfahren erreichen, die zu Partikeln mit hohem Kristallinitätsgrad führen. Weiterhin ist der Zusatz externer, nicht kovalent mit den Partikeln verbundener Stabilisatoren unerwünscht, da sie ausgewaschen werden können oder in vielen Anwendungen, z. B. im Bereich pharmazeutischer Zubereitungen stören.

Aufgabe der vorliegenden Erfindung war es daher neuartige primärpartikulär dispergierbare Nanopartikel auf Cellulose-Basis bereitzustellen, welche sich durch ein vergleichsweise technisch einfach zu realisierendes Verfahren herstellen lassen und zur Partikelbildung nicht auf externe Emulgatoren angewiesen sind.

Es wurde nun gefunden, dass die zugrunde liegende Aufgabe durch Scher- oder Ultraschalldispergierung weitestgehend amorpher Cellulose gelöst werden kann.

Gegenstand der Erfindung sind daher Partikel amorpher Cellulose mit volumengemittelten Partikelgrößen (D50-Wert) von weniger als 200 nm, besonders bevorzugt 100nm, gemessen mittels Ultrazentrifugation oder dynamischer Laserlichtstreumessung und die amorphe Cellulose einen Anteil an kristallinen Bereichen von weniger als 20 Gew.-% aufweist.

Ebenfalls ein Gegenstand der vorliegenden Erfindung ist ein Verfahren zur Herstellung von Dispersionen enthaltend Partikel aus amorpher Cellulose mit volumengemittelten Partikelgrößen (D50-Wert) von weniger als 200 nm, besonders bevorzugt weniger als 100 nm, bestimmt durch Ultrazentrifugation oder dynamische Laserlichtstreuung, bei dem
a) amorphe Cellulose mit einem Anteil an kristallinen Bereichen von weniger als 20 Gew.-% bereitgestellt,
b) anschließend gegebenenfalls zumindest teilweise hydrolysiert und abschließend
c) in Wasser oder einem wasserhaltigen flüssigen Medium aufgenommen und gleichzeitig oder anschließend durch Eintrag von Energie darin dispergiert und gegebenfalls schließlich mit Wasser verdünnt wird.

In einer bevorzugten Ausführungsform der Erfindung beträgt der D90-Wert, und weiter bevorzugt auch der D95-Wert, der amorphen Cellulosepartikel weniger als 200 nm, besonders bevorzugt weniger als 100 nm, wobei die Partikel dabei vorzugsweise agglomeratfrei, d. h. primärpartikulär dispers, vorliegen.

Die Bereitstellung von im Sinne von Schritt a) geeigneten amorphen Cellulosen kann von allen kommerziellen Zellstoffen (z.B. Chemie- und Papierzellstoffe), mikrokristalliner Cellulose oder Linterscellulose ausgehen. Sie besitzen typischerweise Durchschnittspolymerisationsgrade (DP) von 100 bis 3000, bevorzugt 200 bis 2500, bevorzugter 250 bis bis 2000, besonders bevorzugt von 350 bis 1500.

Zur Beseitigung kristalliner Bereiche werden diese Cellulosen dann entweder in einem Lösungsmittel wie N-Methylmorpholin-N-oxid Monohydrat (NMMNO), Salzhydrat-schmelzen, wie z. B. ZnCl₂ + 4H₂O, LiClO₄*3H₂O, FeCl₃*6H₂O oder ionischen Flüssigkeiten, z.B. 1-Butyl-3-Methylimidazoliumchlorid gelöst und anschließend durch Zugabe eines Nicht-Lösungsmittels wieder ausgefällt. Beispiele solcher Nicht-Lösungsmittel sind z.B. Alkohole und Wasser.

Statt des vorbeschriebenen Lösungs-/Fällungsverfahrens zur Bereitstellung amorpher Cellulose kann diese auch durch Mahlung (z.B. in einer Planetenmühle, Kugelmühle) hergestellt werden (P. H. Hermans, A. Weidinger, J. Am. Chem. Soc. 68 (1946) 2547, S. Fischer, Habilitationsschrift, TU Bergakademie Freiberg 2003.).

Bevorzugt liegt der Kristallinitätsgrad der in b) eingesetzten Cellulose bei <20% Der Kristallinitätsgrad wird mit Röntgenweitwinkelstreuung (WAXS) bestimmt.

Die optionale Hydrolyse in Schritt b) kann durch eine Mineralsäure, bevorzugt Schwefelsäure oder Phosphorsäure, oder eine Salzhydratschmelze, bevorzugt Zinkchlorid oder Lithiumperchlorat, bei Temperaturen von 40 °C bis 100 °C vorgenommen werden. Sie kann dazu dienen, die Kettenlänge der Celluloseverbindungen zu verkürzen. Unter den Begriff "Mineralsäure" fallen auch Mineralsäuregemische. Der Begriff "Salzhydratschmelze" umfasst auch Schmelzmischungen.

Bevorzugt wird das Reaktionsgemisch nach der Hydrolyse von darin gelösten Salzen befreit. Dies kann beispielsweise durch Zentrifugation und Wäsche mit Wasser, d.h. durch Abtrennung des Rohprodukts durch Zentrifugation, anschließendem Hinzufügen von Wasser und Abtrennen des Produkts vom Waschwasser durch Zentrifugation, erfolgen. Bevorzugt beträgt der Restsalzgehalt des Hydrolyseprodukts weniger als 5 Gew.-%, besonders bevorzugt weniger als 1 Gew.-%.

Als Medium zur Dispergierung der Partikel wird vorzugsweise Wasser verwendet. Der pH-Wert des nicht stark aciden wässrigen Mediums liegt vorzugsweise im Bereich oberhalb von 5, bevorzugter oberhalb von 6, weiter bevorzugter im Bereich von 6 bis 10, und besonders bevorzugt von 6,5 bis 8.

Der Energieverbrauch für die Dispergierung beträgt mindestens 2000 kWh/t, bevorzugt mindestens 5000 kWh/t, besonders bevorzugt mindestens 10000 kWh/t.

Zum Eintrag von Energie in Schritt c) eignen sich grundsätzlich sämtliche dem Fachmann bekannte Vorrichtungen und Techniken zu diesem Zweck. Bevorzugt ist der Einsatz von Ultraschallgeräten, Hochgeschwindigkeitsrührer, Dispergiereinrichtungen basierend auf dem Rotor-Stator-Prinzip (z.B. Ultra-Turrax-Geräte), Strahldispergatoren und Mikrofluidizer.

Ultra-Turrax-Geräte sind Dispergiereinrichtungen zum Emulgieren, Homogenisieren und Suspendieren fließfähiger Medien. Die wirksame Frequenz ist einstellbar und kann dem zu bearbeitenden Stoff oder Stoffgemisch angepasst werden.

Das Prinzip eines Mikrofluidizer lässt sich wie folgt beschreiben. Das zu verarbeitende Material wird unter hohem Druck durch eine Interaktionskammer geführt. Die Probe strömt durch eine oder zwei schmale Kanäle und erreicht lineare Geschwindigkeiten in Abhängigkeit vom Gerätetyp bis 1000 m/s. So entstehen enorme Scherkräfte. In der Kammer gibt es keine beweglichen Teile, wodurch eine enge Partikel- und Tröpfchenverteilung gewährleistet wird.

Der Energieeintrag in Stufe c) kann grundsätzlich ein- oder mehrstufig aber auch kontinuierlich mit variablem Energieeintrag erfolgen.

Die in Schritt c) erhaltene Dispersion weist eine Feststoffkonzentration der Cellulose von bevorzugt 0,1 bis 10 Gew.-%, besonders bevorzugt 0,5 bis 3,5 Gew.-%, ganz besonders bevorzugt 0,75 bis 2,5 Gew.-%, auf.

Durch die in Schritt c) erfolgende Dispergierung, insbesondere durch den Energieeintrag, kann sich der Polymerisationsgrad der amorphen Cellulose verringern. Dabei ist ein Polymerisationsgradabbau von 5 bis 50% möglich. Infolgedessen kann die in Schritt c) erhaltene nanoskalige amorphe Cellulose einen durchschnittlichen Polymerisationsgrad von 50 bis 2900, bevorzugt 100 bis 2400 aufweisen.

Der Zusatz von Dispersionsmitteln oder Emulgatoren zur Stabilisierung der Celluslosedispersion ist nach dem erfindungsgemäßen Verfahren nicht notwendig.

In einer bevorzugten Ausführungsform der Erfindung wird daher vor, während oder nach dem Dispergierschritt c) kein Dispersionsmittel bzw. Emulgator, vorzugsweise kein Fettsäuresalz oder Alkylbenzolsulfonat, zugesetzt. Gleichwohl kann durch Zusatz solcher Mittel eine weitere Stabilisierung erreicht werden.

Bevorzugt wird das erfindungsgemäße Verfahren, insbesondere Schritt c), bei Temperaturen 10 bis 100 °C, besonders bevorzugt 20 bis 80 °C durchgeführt.

### Beispiele:

Als Cellulosen wurden handelsübliche Holzzellstoffe oder Linterscellulosen eingesetzt.

Zur Dispergierung der Cellulosederivate in Wasser wurde ein Hochgeschwindigkeitsrührer nach dem Rotor-Stator-Prinzip eingesetzt (Ultra Turrax T25 basic, IKA, Umdrehungsgeschwindigkeit 20 000 min⁻¹).

Zur weiteren Homogenisierung wurde ein Mikrofluidizer vom Typ 110F (Microfluidics, Newton MA USA) mit zwei in Reihe geschalteten Interaktionskammern (H210Z 200 µm und JR20Z 50 µm) verwendet.

Die Röntgenweitwinkelmessungen wurden am Zweikreis-Diffraktometer D5000 der Fa. Bruker-AXS in symmetrischer Transmission unter Verwendung monochromatischer Cu-Kα-Strahlung (Ge(111)-Monochromator) durchgeführt. Die Aufnahmen erfolgten bei 30 mA und 40 kV in dem 20-Winkelbereich von 4-104° (Schrittweite Δ2θ = 0.2°). Aus den Diffraktometerstreukurven wurde mit Hilfe der IAP-Auswertesoftware WAXS 7 (Methode nach Ruland-Vonk) ein Kristallinitätsgrad x_{c} ermittelt.

Die analytische Ultrazentrifugation erfolgte mit Hilfe des Geräts OPTIMA XL-I der Fa. Beckman-Coulter (Palo Alto, CA) unter Verwendung von Doppelsektor-Zellen mit 12 mm optischer Weglänge und Interferenz-Optik als Detektor. Messungen der Sedimentationsgeschwindigkeit wurden bei Drehzahlen von 15.000 U/min bei jeweils vier verschiedenen Konzentrationen zwischen 0,3 und 0,05% bei 20 °C durchgeführt. Die aus den Sedimentogrammen ermittelten mittleren Sedimentationskoeffizienten wurden gemäß 1/sapp = 1/so + (ks/so)·c auf die Konzentration c=0 extrapoliert.

Die dynamische Laserlichtstreumessung erfolgte mit dem Gerät Horiba LB 550 (USA) mit einem Messbereich von 1 nm bis 6 µm. Dazu werden die Diffusionsgeschwindigkeiten der dispergierten Partikel über die Doppler-Verschiebung in der Frequenz des an ihnen gestreuten Laserlichts gemessen. Die Frequenzverschiebungen werden als Intensitätsschwankungen des Streulichtes an einem Detektor erfasst. Es werden sowohl die D50 (50% der Teilchen sind kleiner als die angegebene Dimension) als auch die D90- Werte (90% der Teilchen sind kleiner als die angegebene Dimension) bestimmt.

### Referenzbeispiel 1:

### a) Bereitstellung amorpher Cellulose

53 g Cellulose (Durchschnittpolymerisationsgrad DP_{cuoxam (Tetramminkupfer(II)hydroxidlösung)} = 751, Kristallinitätsgrad x_{c} = 48%) wurden unter Zusatz von 0,75 Gallussäurepropylester in 2800 g einer 46%igen N-Methylmorpholin-N-oxid-Lösung (NMMNO) suspendiert. Bei einer Temperatur von 105 °C und 60 mbar wurden 1320 g Wasser abgedampft, wobei sich die Cellulose auflöste. Zu dieser Lösung wurden schrittweise bei 80 °C 1,5 Liter 2-Propanol gegeben, wobei die Cellulose ausfiel. Das Produkt wurde abgetrennt, mit 2-Propanol gewaschen und im Vakuumtrockenschrank getrocknet. Die erhaltene Cellulose ist amorph. Der DP dieser Probe betrug DP_{cuoxam}= 657.

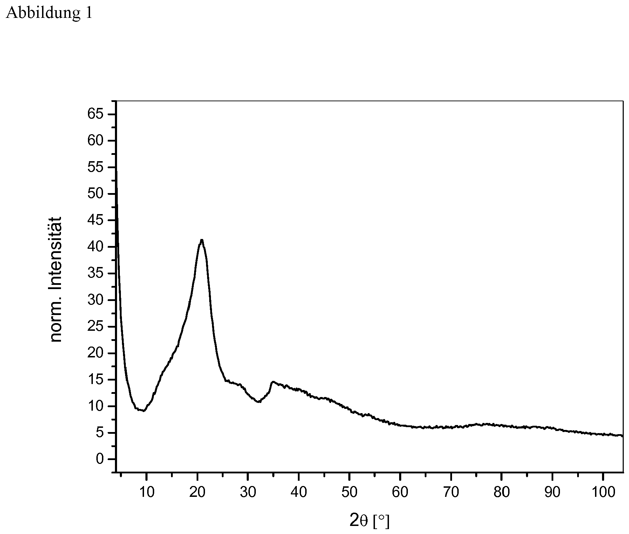

Abbildung 1 zeigt die Streukurve der erhaltenen amorphen Cellulose. Der daraus ermittelte Kristallinitätsgrad x_{c} beträgt 14%.

### b) Herstellung einer Dispersion nanoskaliger Cellulosepartikel

10 g dieser amorphen Cellulose wurden in 490 ml 20 Gew.-%iger Schwefelsäure bei 80 °C und 6 h hydrolysiert. Dann wurde die Suspension in 1 Liter Wasser unter Verwendung eines Ultra-Turrax eingebracht und mittels einer Zentrifuge bis zur pH-Neutralität und Salzfreiheit gewaschen.

Zur Dispergierung wurden 300 ml einer 2 Gew.-%igen Suspension dieser Cellulose in Wasser zunächst für 30 min mit einem Ultra-Turrax und anschließend mit einem Mikrofluidizer jeweils eine Stunde bei 600 bar und 1100 bar behandelt. Nach der Mikrofluidizer-Behandlung wird die Feststoff-Konzentration durch Zugabe von Wasser auf 1,5 Gew.-% eingestellt.

Im Ergebnis erhielt man eine völlig transparente, stabile und opaleszierende Dispersion von Cellulose mit einem DP_{cuoxam} = 52.

Die Probe wurde mittels Ultrazentrifugation untersucht.

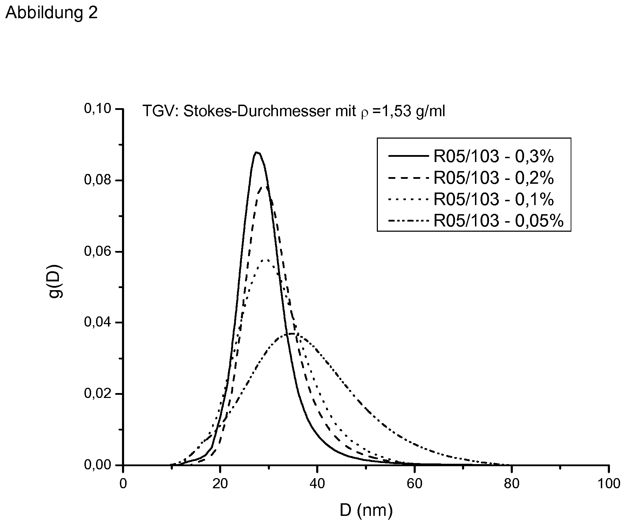

Abbildung 2 und Tabelle 1 zeigen, dass die mittlere Partikelgröße ohne Quellungskorrektur bei 37,3 nm lag.

**Tabelle 1:**

| Probe | Fläche | Zentrum | Breite | Offset | Höhe |
|---|---|---|---|---|---|
| 0,3 % | 0,9258 | **28,217** | 8,7553 | 0,00105 | 0,084371 |
| 0,2 % | 0,9319 | **29,777** | 10,020 | 0,001124 | 0,07420 |
| 0,1 % | 0,9836 | **30,201** | 14,262 | 4,160x10⁻⁴ | 0,055027 |
| 0,05 % | 0,9353 | **35,862** | 21,800 | 0,00131 | 0,034232 |

| | | | | | |
|---|---|---|---|---|---|
| D₀ = 37,3 nm (berechnet aus s₀ =407,1 Svedbg.) | | | | | |

Aus dem Sedimentationsverhalten konnte man auf eine Quellung der Partikel in Folge einer fest gebundenen Hydrathülle schließen. Unter Berücksichtigung der Quellung der Teilchen betrug die Teilchengröße 120 bis 190 nm.

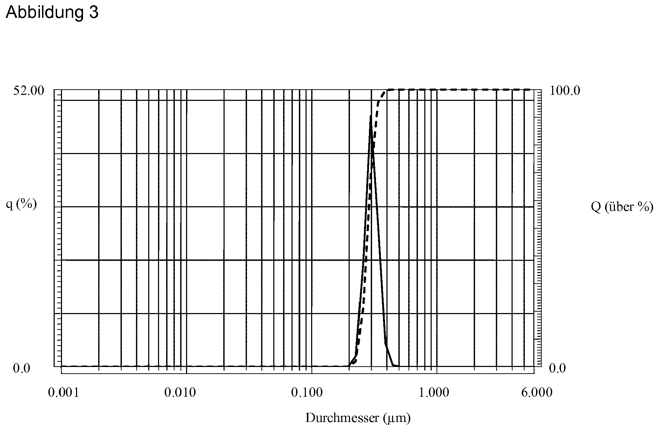

Abbildung 3 zeigt die dynamische Laserlichtstreumessung der Nanocellulose-Dispersion hergestellt nach Beispiel 1 (0,08 Gew.-%, 2 min Ultraschall). Es ergibt sich ein D50-Wert von 281 nm sowie ein D90-Wert von 330 nm.

## Patentansprüche

1. Verfahren zur Herstellung einer Dispersion enthaltend Partikel aus amorpher Cellulose bei dem
a) amorphe Cellulose mit einem Anteil an kristallinen Bereichen von weniger als 20 Gew.-% bereitgestellt,
b) anschließend gegebenenfalls zumindest teilweise hydrolysiert, und
c) danach in Wasser oder einem wasserhaltigen flüssigen Medium aufgenommen und gleichzeitig oder anschließend durch Eintrag von Energie darin dispergiert und gegebenenfalls schließlich mit Wasser verdünnt wird,
wobei die volumengemittelte Größe der Teilchen (D50-Wert) weniger als 200 nm, besonders bevorzugt 100 nm, bestimmt durch Ultrazentrifugation oder dynamische Laserlichtstreuung, beträgt, wobei die kristallinen Bereiche mittels Röntgenweitwinkelmessungen bei einem 20-Winkelbereich von 4-104° nach der Methode nach Ruland-Vonk bestimmt werden.

2. Verfahren nach Anspruch 1, wobei der D90-Wert, und bevorzugt der D95-Wert, der dispergierten Partikel weniger als 200 nm, bevorzugter weniger als 100 nm, beträgt.

3. Verfahren nach Anspruch 1 oder 2, wobei die amorphe Cellulose aus Chemiezellstoff, Papierzellstoff, mikrokristalliner Cellulose, oder Linterscellulose gewonnen wird.

4. Verfahren nach einem der vorherigen Ansprüche, wobei die amorphe Cellulose einen durchschnittlichen Polymerisationsgrad von 100 bis 3000, bevorzugt 200 bis 2500, bevorzugter 250 bis 2000, besonders bevorzugt 350 bis 1500, aufweist.

5. Verfahren nach einem der vorherigen Ansprüche, wobei in Schritt a) die amorphe Cellulose mit einem Anteil an kristallinen Bereichen von weniger als 20 Gew.-% dadurch bereitgestellt wird, dass die Ausgangscellulose gemahlen wird oder in einem Lösungsmittel gelöst und anschließend durch Zugabe eines Nicht-Lösungsmittels wieder ausgefällt wird.

6. Verfahren nach Anspruch 5, wobei das Lösungsmittel N-Methylmorpholin-N-oxid Monohydrat, eine Salzhydratschmelze oder eine ionische Flüssigkeit ist und/oder das Nicht-Lösungsmittel ein Alkohol, Wasser oder Mischungen davon ist.

7. Verfahren nach einem der vorherigen Ansprüche, wobei die Hydrolyse in Schritt b) durch eine Mineralsäure oder Salzhydratschmelze, vorzugsweise bei Temperaturen zwischen 40 °C und 100 °C, vorgenommen wird.

8. Verfahren nach einem der vorherigen Ansprüche, wobei nach der Hydrolyse das Reaktionsgemisch von darin gelösten Salzen befreit wird, vorzugsweise durch Zentrifugation und Wäsche mit Wasser, und der Restsalzgehalt des Hydrolyseprodukts bevorzugt weniger als 5 Gew.-% beträgt.

9. Verfahren nach einem der vorherigen Ansprüche, wobei in Schritt c) mindestens 2000 kWh/t, bevorzugt mindestens 5000 kWh/t, besonders bevorzugt mindestens 10000 kWh/t, Energie eingetragen wird.

10. Verfahren nach einem der vorherigen Ansprüche, wobei der Eintrag von Energie in Schritt c) durch Ultraschallgeräte, Hochgeschwindigkeitsrührer, Dispergiereinrichtungen basierend auf dem Rotor-Stator-Prinzip, Strahldispergatoren oder Mikrofluidizern erfolgt.

11. Verfahren nach einem der vorherigen Ansprüche, wobei die in Schritt c) erhaltene Dispersion eine Feststoffkonzentration der Cellulose von 0,1 bis 10 Gew.-%, bevorzugt 0,5 bis 3,5 Gew.-%, besonders bevorzugt 0,75 bis 2,5 Gew.-%, aufweist.

12. Verfahren nach einem der vorherigen Ansprüche, wobei vor, während oder nach dem Dispergierschritt c) kein Dispersionsmittel, vorzugsweise kein Fettsäuresalz oder Alkylbenzolsulfonat, zugesetzt wird.

13. Verfahren nach einem der vorherigen Ansprüche, wobei Schritt c), bei Temperaturen 10 bis 100 °C, bevorzugt bei 20 bis 80 °C durchgeführt wird.

14. Dispersion enthaltend Partikel aus amorpher Cellulose, wobei die volumengemittelte Größe der Partikel (D50-Wert) weniger als 200 nm, besonders bevorzugt 100 nm, bestimmt durch Ultrazentrifugation oder dynamische Laserlichtstreuung, beträgt und die amorphe Cellulose einen Anteil an kristallinen Bereichen von weniger als 20 Gew.-% aufweist, wobei die kristallinen Bereiche mittels Röntgenweitwinkelmessungen bei einem 20-Winkelbereich von 4-104° nach der Methode nach Ruland-Vonk bestimmt werden.

15. Dispersion nach Anspruch 14, wobei die amorphe Cellulose aus Chemiezellstoff, Papierzellstoff, mikrokristalliner Cellulose, oder Linterscellulose gewonnen wird.

16. Dispersion nach einem der Ansprüche 14 oder 15, wobei die amorphe Cellulose einen durchschnittlichen Polymerisationsgrad von 50 bis 2900, bevorzugt 100 bis 2400 aufweist.

17. Dispersion nach einem der Ansprüche 14 bis 16, wobei in der Dispersion die Feststoffkonzentration der Cellulose 0,1 bis 10 Gew.-%, bevorzugt 0,5 bis 3,5 Gew.-%, beträgt.

18. Dispersion erhältlich durch ein Verfahren nach einem der Ansprüche 1 bis 13.

19. Partikel aus amorpher Cellulose wie sie in der Dispersion nach einem der Ansprüche 14 bis 18 enthalten sind.

## Claims

1. Process for producing a dispersion containing particles of amorphous cellulose, which process comprises
a) amorphous cellulose having a less than 20% by weight fraction of crystalline regions being provided,
b) subsequently, if appropriate, being at least partially hydrolysed, and
c) thereafter being taken up in water or a water-containing liquid medium and being simultaneously or subsequently dispersed therein by input of energy and finally, if appropriate, being diluted with water,
wherein the volume-average particle size (D50 value) of the particles is less than 200 nm, preferably less than 100 nm, determined by ultracentrifucation or dynamic laser light scattering, wherein the crystalline ranges are determined by wide angle X-ray measurements at an 2θ angle range of 4 to 104° according to the method of Ruland-Vonk.

2. Process according to claim 1, wherein the D90 value, preferably the D95 value of the dispersed particles is less than 200 nm, preferably less than 100 nm.

3. Process according to claim 1 or 2, wherein the amorphous cellulose used is obtained from chemical pulp, paper-grade pulp, microcrystalline cellulose or linters cellulose.

4. Process according to any of the preceding claims, wherein the amorphous cellulose used has an average degree of polymerization in the range from 100 to 3000, preferably 200 to 2500, more preferably 250 to 2000 and most preferably 250 to 1500.

5. Process according to any of the preceding claims, wherein in step a) the amorphous cellulose having a less than 20 % by weight fraction of crystalline regions is provided by the starting cellulose being ground or being dissolved in a solvent and subsequently reprecipitated by addition of a non-solvent.

6. Process according to claim 5, wherein the solvent is N-methylmorpholine N-oxide monohydrate, a salt hydrate melt or an ionic liquid and/or the non-solvent is an alcohol, water or mixtures thereof.

7. Process according to any of the preceding claims, wherein the hydrolysis in step b) is effected by means of a mineral acid or salt hydrate melt, preferably at temperatures between 40° C and 100° C.

8. Process according to any of the preceding claims, wherein after hydrolysis the reaction mixture is freed of salts dissolved therein, preferably by centrifugation and washing with water, and the residual salt content of the hydrolysis product is preferably less than 5 % by weight.

9. Process according to any of the preceding claims, wherein the energy input in step c) is at least 2000 kWh/t, preferably at least 5000 kWh/t, more preferably at least 10000 kWh/t.

10. Process according to any of the preceding claims, wherein the input of energy in step c) is effected via ultrasonicators, high-speed stirrers, dispersing devices based on the rotor-stator principle, jet dispersers or microfluidizers.

11. Process according to any of the preceding claims, wherein the dispersion obtained in step c) has a solids concentration of the cellulose in the range from 0.1 % to 10 % by weight, preferably from 0.5 % to 3.5 % by weight, more preferably from 0.75 % to 2.5 % by weight.

12. Process according to any of the preceding claims, wherein no dispersant, preferably no fatty acid salt or alkylbenzenesulphonate is added before, during or after the dispersing step c).

13. Process according to claim 1, wherein step c) is carried out at temperatures of 10 to 100° C, preferably 20 to 80 °C.

14. Dispersion containing particles of amorphous cellulose, wherein the volume-average particle size (D50 value) is less than 200 nm, preferably 100 nm, determined by ultracentrifugation or dynamic laser light scattering, and wherein the amorphous cellulose has a less than 20% by weight fraction of crystalline regions, wherein the crystalline regions are determined by wide angle X-ray measurements at an 2θ angle range of 4 to 104° according to the method of Ruland-Vonk..

15. Dispersion according to claim 14, wherein the amorphous cellulose is obtained from chemical pulp, paper-grade pulp, microcrystalline cellulose or linters cellulose.

16. Dispersion according to any of claims 14 or 15, wherein the amorphous cellulose has an average degree of polymerization in the range from 50 to 2900, preferably from 100 to 2400.

17. Dispersion according to any of claims 14 to 16, wherein the solids concentration of the cellulose in the dispersion is in the range from 0.1 % to 10 % by weight, preferably 0.5 to 3.5 % by weight.

18. Dispersion obtainable by a process according to any of claims 1 to 13.

19. Particles of amorphous cellulose as present in the dispersion according to any of claims 14 to 18.

## Revendications

1. Procédé de préparation d'une dispersion contenant des particules de cellulose amorphe dans lequel
a) de la cellulose amorphe, pourvue d'une proportion de zones cristallines inférieure à 20 % en poids est préparée,
b) si nécessaire, elle est hydrolysée par la suite au moins partiellement et
c) est ensuite absorbée dans de l'eau ou un milieu liquide aqueux et dispersée simultanément ou ultérieurement par apport d'énergie, puis, si nécessaire, est diluée avec de l'eau,
la taille moyenne en volume des particules (valeur D50) étant inférieure à 200 nm, de manière particulièrement préférée à 100 nm, détermination faite par ultracentrifugation ou diffusion dynamique de la lumière laser, les zones cristallines étant déterminées au moyen de mesures aux grands angles par rayons X dans une plage angulaire 2θ de 4 à 104° selon la méthode de Ruland-Vonk.

2. Procédé selon la revendication 1, la valeur D90, et de préférence la valeur D95, des particules dispersées étant inférieure à 200 nm, plus préférablement à 100 nm.

3. Procédé selon la revendication 1 ou 2, la cellulose amorphe étant obtenue à partir de pâte chimique, de pâte à papier, de cellulose microcristalline ou de cellulose de linters.

4. Procédé selon l'une des revendications précédentes, la cellulose amorphe possédant un degré de polymérisation moyen de 100 à 3 000, de préférence de 200 à 2 500, plus préférablement de 250 à 2 000, de manière particulièrement préférée de 350 à 1500.

5. Procédé selon l'une des revendications précédentes, sachant qu'à l'étape a) la cellulose amorphe pourvue d'une proportion de zones cristallines inférieure à 20 % en poids est préparée par le fait que la cellulose de départ est broyée ou est dissoute dans un solvant, puis est à nouveau précipitée par ajout d'un non-solvant.

6. Procédé selon la revendication 5, le solvant étant un monohydrate de N-oxyde de N-méthylmorpholine, une masse fondue d'hydrates de sel ou un liquide ionique et/ou le non-solvant étant un alcool, de l'eau ou des mélanges de ceux-ci.

7. Procédé selon l'une des revendications précédentes, l'hydrolyse à l'étape b) s'effectuant par un acide minéral ou une masse fondue d'hydrates de sel, de préférence à des températures comprises entre 40 °C et 100 °C.

8. Procédé selon l'une des revendications précédentes, sachant qu'après l'hydrolyse, le mélange réactionnel est libéré des sels dissous dans ce dernier, de préférence par centrifugation et lavage à l'eau, et la teneur en sel résiduel du produit d'hydrolyse est de préférence inférieure à 5 % en poids.

9. Procédé selon l'une des revendications précédentes, sachant qu'à l'étape c) il y a un apport énergétique d'au moins 2 000 kWh/t, de préférence d'au moins 5 000 kWh/t, de manière particulièrement préférée d'au moins 10 000 kWh/t.

10. Procédé selon l'une des revendications précédentes, l'apport énergétique à l'étape c) s'effectuant au moyen d'appareils à ultrasons, d'agitateurs à grande vitesse, de dispositifs de dispersion basés sur le principe rotor-stator, de diffuseurs à jet ou de microfluidiseurs.

11. Procédé selon l'une des revendications précédentes, la dispersion obtenue à l'étape c) possédant une concentration en solides de cellulose de 0,1 à 10 % en poids, de préférence de 0,5 à 3,5 % en poids, de manière particulièrement préférée de 0,75 à 2,5 % en poids

12. Procédé selon l'une des revendications précédentes, aucun agent de dispersion, de préférence aucun sel d'acide gras ou sulfonate d'alkylbenzène n'étant ajouté avant, pendant ou après l'étape de dispersion c).

13. Procédé selon l'une des revendications précédentes, l'étape c), étant effectuée à des températures comprises entre 10 à 100 °C, de préférence entre 20 et 80 °C.

14. Dispersion contenant des particules de cellulose amorphe, la taille moyenne en volume des particules (valeur D50) étant inférieure à 200 nm, de manière particulièrement préférée à 100 nm, détermination faite par ultracentrifugation ou diffusion dynamique de la lumière laser, et la cellulose amorphe possédant une proportion de zones cristallines inférieure à 20 % en poids, les zones cristallines étant déterminées au moyen de mesures aux grands angles par rayons X dans une plage angulaire 2θ de 4 à 104° selon la méthode de Ruland-Vonk.

15. Dispersion selon la revendication 14, la cellulose amorphe étant obtenue à partir de pâte chimique, de pâte à papier, de cellulose microcristalline ou de cellulose de linters.

16. Dispersion selon l'une des revendications 14 ou 15, la cellulose amorphe possédant un degré de polymérisation moyen de 50 à 2 900, de préférence de 100 à 2 400.

17. Dispersion selon l'une des revendications 14 à 16, la concentration en solides de cellulose dans la dispersion allant de 0,1 à 10 % en poids, de préférence de 0,5 à 3,5 % en poids.

18. Dispersion pouvant être obtenue par un procédé selon l'une des revendications 1 à 13.

19. Particules de cellulose amorphe comme celles contenues dans la dispersion selon l'une des revendications 14 à 18.
